# EUROPEAN PATENT APPLICATION

(11) **EP 3 610 835 A1**
(43) Date of publication of application: **19.02.2020**
(21) Application number: 18189624.2
(22) Date of filing: 17.08.2018
(51) Int. Cl.: A61F 9/00

(54) **CONTACT LENS APPLICATOR**

(71) Applicant: Optic 18 Limited, Leicestershire LE11 3PE (GB)
(72) Inventor: The designation of the inventor has not yet been filed
(74) Representative: Browne, Robin Forsythe

(57) **Abstract**

A contact lens applicator comprising: a support (12) for a contact lens and a handle (11); the support having an axis of symmetry extending vertically in use; the handle extending radially from the support; the handle being generally laminar and having upper and lower surfaces; the support comprising an annular support body surrounding an axial drainage port (4), the support body having upper and lower surfaces (5,6); wherein the upper surface of the support body is convex; wherein one or both of the upper and lower surfaces of the handle comprises a projections (20,21) configured to facilitate use of the handle as a contact lens removal device by drawing of the surface across the in situ contact lens.

## Description

This invention relates to an applicator which may be used to place a contact lens onto a wearer's eye.

Experienced wearers of contact lenses are practiced at inserting a contact lens onto their eyeball and removal from the eye using the tip of a finger as it approaches the eye. In order to do so a user needs to look directly at the tip of his or her finger. This may be difficult for new users or for users who are infirm. Such difficulty may lead to abandonment of the use of contact lenses and may contribute to a low uptake of contact lenses by aspiring users.

Removal of contact lenses may be also problematic, particularly as a lens may adhere strongly if a wearer is dehydrated, for example following exercise. Removal using a fingertip can be uncomfortable, may damage the lens or a wearer's eyeball and can cause hygiene issues.

EP 3045071 discloses a contact lens dispensing container comprising;
an upwardly opening receptacle configured to receive a single contact lens and storage solution, the container having a peripheral flange, a peelable cover sheet and an applicator comprising a handle pivotally mounted on the flange.

WO 2017/220684 discloses a contact lens dispensing container comprising;
an upwardly opening receptacle configured to receive a single contact lens, an applicator comprising a lens support, a handle and a peelable cover sheet.

According to a first aspect of the invention, a contact lens applicator comprises a support for a contact lens and a handle;
the support having an axis of symmetry extending vertically in use;
the handle extending radially from the support;
the handle being generally laminar and having upper and lower surfaces;
the support comprising an annular support body surrounding an axial drainage port, the support body having upper and lower surfaces;
wherein the upper surface of the support body is convex;
wherein one or both of the upper and lower surfaces of the handle are provided with a relatively high friction surface configured to facilitate use of the handle as a contact lens removal device by drawing of the surface across an in situ contact lens.

According to a second aspect of the present invention, a contact lens applicator comprises a support for a contact lens and a handle;
the support having an axis of symmetry extending vertically in use;
the handle extending radially from the support;
the handle being generally laminar and having upper and lower surfaces;
the support comprising an annular support body surrounding an axial drainage port, the support body having upper and lower surfaces;
wherein the upper surface of the support body is convex; and
wherein upper and lower surfaces are configured so that the lower surface of the applicator may engage with an upper surface of a second identical applicator to form a stacked arrangement.

In embodiments features of each aspect of this invention may be employed individually or combined.

In embodiments three or more identical applicators may be located in stacked relation to form a nested arrangement. The applicators may be stacked vertically or horizontally. Such an arrangement facilitates storage and transportation of a quantity of applicators for daily use. A plurality of applicators may be provided in stacked relation in a container such as a case having a tray closed by a lid cover.

Alternatively or in addition a plurality of identical applicators may be joined by frangible connections to form a strip of applicators, each individually applicator being manually detachable from an adjacent applicator for use. The strips may be stackable or nestable. Provision of a stacked arrangement of individual applicators or strips confers the advantage that an underlying applicator may be covered and protected from ingression of debris and thereby kept clean and sterile.

The applicator may be employed by a user holding the handle to apply the contact lens to the eyeball. A user may use the applicator to scoop and raise a lens from a liquid storage receptacle, the liquid draining from the lens through the drainage port leaving the lens centred on the upper surface of the annular support. During application to the eye, a user may turn his or her face downwardly, raising the applicator upwardly towards the eyeball to lift a contact lens supported upon the support into engagement with the eye. Insertion from the side is facilitated. Visibility when a user is looking into a mirror in provided. These may be a major advantages to a user, particularly to an inexperienced or infirm user who may have difficulty in coordination.

The support body may be toroidal in shape and may be circular in cross-section

In an alternative embodiment, the lower surface of the support body is flattened, inclined or concave to facilitate engagement with or stacking upon a complementary upper surface of an underlying identical applicator.

The surface may be configured to facilitate use for lens removal as well as an applicator, by comprising one or more of: one or more raised projections, relatively high friction regions or coatings, one or more regions or coatings having tack properties when in contact with a surface of a contact lens.

The lower surface may be provided with one or more, preferably three or more, downwardly extending bosses. The bosses may serve to prevent inadvertent pick-up of a lens by the underside of a support in use. A flattened or concave surface may further facilitate resting of the applicator on a horizontal surface such as a table top or bathroom unit.

The upper surface of the support may be smooth, provided with raised features or projections or may comprise drainage channels to facilitate drainage of storage solution and to prevent unwanted adhesion of a lens to the upper surface.

The handle may extend radially from the support in a direction perpendicular to the axis of the support.

Alternatively, the handle may extend radially in a downwardly or upwardly direction in use from the support so that an end of the handle remote from the support is located below or above the support in use. Such an arrangement facilitates manual engagement of the applicator by a user when the support is approaching a user's eye. In an advantageous embodiment the handle extends downwardly from the support in a direction which may be selected to facilitate alignment of a lens on the support with the eye in use by touching a user's face, for example the eye socket by the user's fingers or thumb while holding the handle.

In an advantageous embodiment, the handle has a first portion connected by a neck to the support and extending a predetermined distance from the axis, the first portion curving upwardly or extending at an angle, preferably greater than 30°, more preferably greater than 45°, to the horizontal and terminating in an alignment region adjoining a manual engagement region. The radial location of the alignment region relative to the upper surface of the support may be selected so that the alignment portion is in contact with or in closely spaced relation to a user's eye socket or orbit, for example in a direction extending horizontally across the user's face. This enables an inexperienced or infirm user to align a contact lens located on the support correctly in relation to the eye by contacting the alignment region with his or her fingers while holding the manual engagement region so that the fingers touch the edge of the eye socket at a location horizontally in relation to the pupil of the eye. The horizontal direction may extend from the eye generally towards a user's ear.

The manual engagement portion of the handle may extend radially outwardly from the alignment portion, for example at a relatively shallow angle or horizontally in comparison to the first portion of the handle. The manual engagement portion may be provided with one or more raised finger grip projections, a roughened surface or high friction surface features. Any other convenient location on the face may be used.

In an embodiment a kit may be provided, the kit comprising a plurality of applicators with handles of different lengths, so that a user may select an applicator with an appropriate length of handle to suit the dimensions of his or her face.

In an advantageous embodiment the handle is generally laminar, for example having flat or generally planar upper and lower surfaces.

A laminar configuration may be advantageous to facilitate stacking and convenient removal of a single applicator or strip thereof from a stack prior to use.

One or both of the upper and lower surfaces may be provided with an array of projections which extend upwardly or downwardly respectively. The projections may facilitate gripping and secure engagement by a user's fingers. The respective arrays may be configured so that the downwardly extending projections of the lower surface of a handle may engage or interlock between or adjacent corresponding upwardly extending projections of an underlying identical applicator handle when the applicators are located in stacked relation.

One or both of the upper and lower surfaces may have a peripheral projection, beading or flange. The flange may extend the whole length or along part of the perimeter of the handle. This may avoid the presence of a sharp edge and may reduce the likelihood that the handle may slip from a user's fingers in use and may facilitate use of the applicator for contact lens removal, by providing a peripheral region defining a maximum area of contact region available to engage the surface of a lens applied to a user's eye.

Some or all of the projections may be provided with a coating of a relatively high friction material, such as an elastomeric polymer or latex. The use of a high friction material may further serve to reduce the likelihood of slippage of the handle from a user's hand in use. The friction material or other coating may be applied conveniently by dip molding.

In the second aspect of the present invention, the applicator may further comprise a longitudinally symmetrical formation adapted to engage and facilitate removal of a lens from a user's eye by drawing the handle across the user's eye. The handle of the applicator may be adapted for use as an aid to removal of the contact lens from a user's eye. The manual engagement portion of the handle may comprise a surface coated with the high friction material.

One or both surfaces of the handle may be planar or substantially flat so that the upper and lower surfaces of the handle may be drawn across the user's eye to facilitate removal of a contact lens. The outer manual engagement portion or end part of the handle may be flexible or resiliently deformable. In an embodiment the handle may have a core of relatively rigid material and a soft tip composed of flexibly deformable material.

The projections may be curved or angled in relation to a radial dimension of the handle to facilitate engagement with the surface of a contact lens.

The projections or the entire upper or lower surface may be coated with a bactericidal composition, for example particulate or nanoparticulate silver, for example applied as a silver halide composition.

The applicator may be completely or partially composed of a polymeric material selected to develop an ionic charge to create an attraction force between the lens and carrier during or after drainage of the storage solution after removal of the applicator from a storage receptacle. For example a partial coating of the material may be employed.

The solution used for storage of the contact lens material may be a saline and or surfactant-containing solution. The applicator may be adapted for use with any commercially available solution.

Development of an ionic charge may serve to facilitate development of an attraction force between a lens and support. Alternatively or in addition the charge may serve to retain the lens in a desired location and facilitate centreing of the lens upon the upper surface of the support and retention of the lens in the correct orientation during and after drainage of the storage liquid.

Contact lenses are transparent and may be difficult for a user to see and locate prior to removal from the storage solution. Therefore correct orientation may be difficult to achieve, resulting in a risk of the lens becoming dislodged and falling from the applicator.

The applicator may be composed of a biodegradable thermoplastic, for example polycaprolactone or polyactic acid or a co-polymer or blend thereof. Accordingly the applicator of the present invention may be conveniently disposable by flushing without adverse environmental consequences. The material may be dyed or pigmented, for example with a readily seen colour, such a high visibility 555nm yellow colour.

Contact lenses may be negatively charged after manufacture. The negatively charged surfaces, for example the upper surface of the support, may exhibit the disadvantage of attracting protein deposition. The whole or part of the applicator may be coated with or composed partially or entirely from a silicone hydrogel. Silicone hydrogels may exhibit a positive charge, facilitating attraction of a contact lens to the applicator during removal from the storage solution and application to a user's eye.

An applicator in accordance with the present invention may confer several advantages. The applicator is simple and easy to use facilitating application of a contact lens to the eye by an inexperienced or infirm user. Use of the applicator may improve user compliance, especially for inexperienced users who may have difficulty in applying a lens using a fingertip inserted directly into the eye. Use of the applicator is hygienic, reducing the risk of ocular contamination or infection. The applicator may be disposable, for example by flushing, being readily biodegradable.

In particularly advantageous embodiments the applicator may additionally be used to facilitate removal of the contact lens. This feature avoids the need for a separate contact lens removal device.

The invention is further described by means of example, but not in any limitative sense, with reference to the accompanying drawings, of which:
Figures 1(a)-(e) are views of a first embodiment of the invention;
Figures 2(a)-(e) are views of a second embodiment of the invention; and
Figures 3(s)-(e) are views of a third embodiment of the invention.

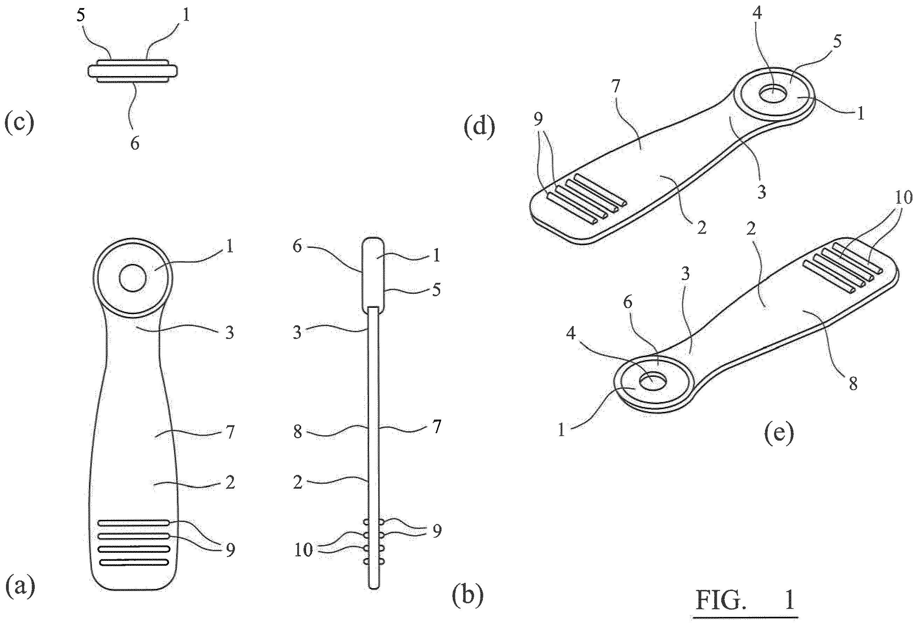

Figures 1(a)-(e) shows various views of a first applicator in accordance with this invention. The applicator comprises a support (1) and handle (2) connected to the support by a neck (3).

The support (1) is generally toroidal, having an axial drainage port (4). The axis extends vertically in use of the applicator. The handle extends radially from the support in a direction perpendicular to the axis, in a horizontal direction in use. The upper surface (5) of the support has a convex annular surface, the diameter and radius of curvature being selected to allow the outer surface of a contact lens to be supported on the convex annular surface, permitting storage liquid to drain from the lens and convex surface during and after removal from the storage receptacle.

The lower surface (6) of the support is also convex in the illustrated embodiment so that the applicator is bilaterally symmetrical, but may be differently shaped in accordance with the embodiments described below.

The handle (2) is laminar having flat upper and lower generally planar surfaces. An array of parallel transversely extending projections (9,10) is provided on each of the upper and lower surfaces. A peripheral flange extends around the edge of the handle to provide a rounded edge to reduce any likelihood of damage to a user's eye.

The applicator may be constructed from the following materials [DETAILS]

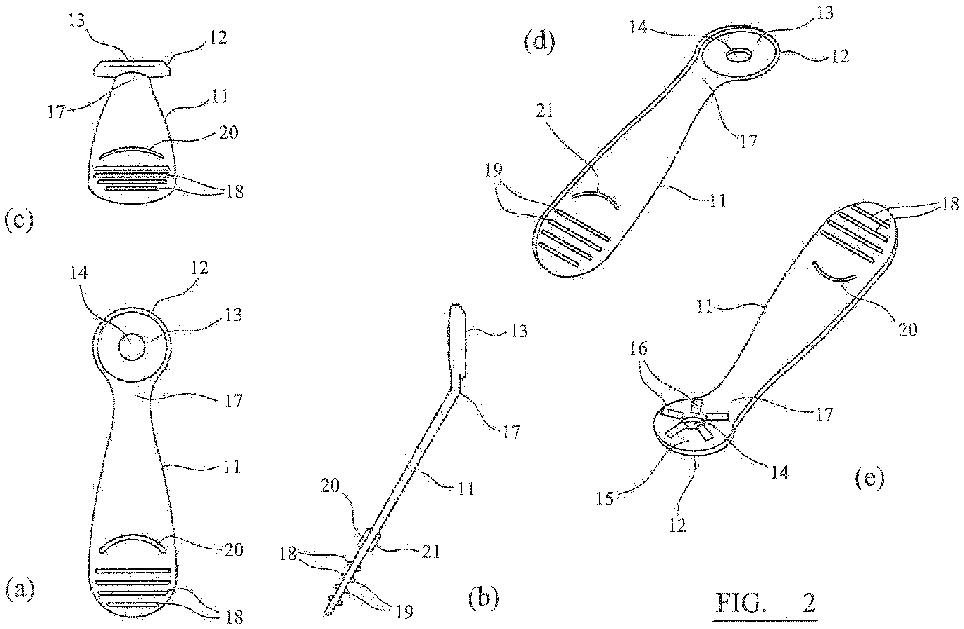

Figures 2(a)-(e) show an alternative applicator in which the handle (11) extends from the support (12) at an angle relative to the axis of the support (12).

The support (12) has a convex upper surface (13) and an axial drainage port (14). The lower surface (15) is generally flattened and has a plurality, for example 5, bosses extending from the surface (15). The bosses prevent accidental picking up of a contact lens by the lower surface in use.

The handle (11) is connected to the support (12) by a neck (17) of reduced transverse dimension. The handle is generally straight, extending downwardly from the neck (17) at a convenient angle, for example about 30°. The angle and length of the handle may be selected so that a user can conveniently hold the applicator when applying the lens to the eyeball, with the hand close to but not contacting the face.

The proximal end of the handle is provided on both the upper and lower sides with an array of parallel transversely extending projections (18,19).

Additional projections (20,21) facilitate use of the handle to remove a lens from a user's eye by drawing the handle across the outer surface of the lens. The curved configuration of the projections (20,21) is configured to conform to the curvature of the external surface of a lens, increasing the engagement force applied to the lens and reducing the likelihood of unwanted sideways movement of the lens during removal.

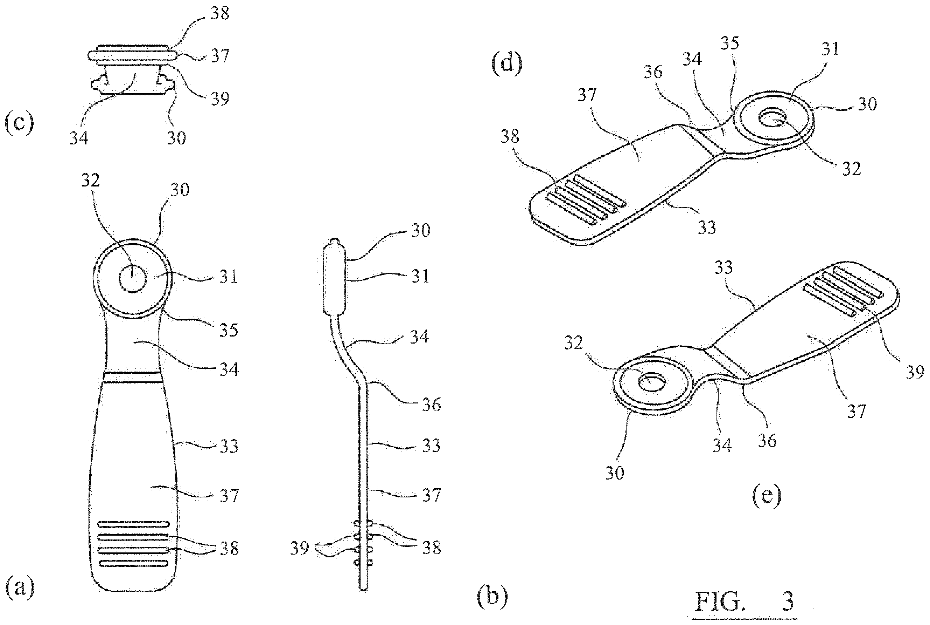

Figures 3(a)-(e) show a further embodiment. A support (30) has a convex upper surface (31) and a drainage port (32), the support having an axis of symmetry which extends vertically in use.

The handle (33) comprises a first portion (34) connected to the support (30) by a neck (35). The proximal end of the first part comprises an alignment region (36). A manual engagement region (37) of the handle extends proximally from the alignment region.

The handle is generally laminar and extends radially from the support. The first portion (34) is curved, having a neck which adjoins the support in a horizontal direction. The first portion curves upwardly to an angle of, for example, 30° to adjoin the alignment region (36). The manual engagement portion (37) extends from the alignment region in a generally horizontal direction. The handle has an array of upwardly and downwardly extending transverse projections (38,39) as disclosed in relation to Figures 1 and 2.

In use a user may hold the engagement portion of the handle with a finger and thumb with the finger and thumb abutting the alignment region. The correct positioning of the lens mounted on the support of the applicator is determined by contacting the user's finger or thumb against the eye socket or other convenient portion of the face.

## Claims

1. A contact lens applicator comprising:
a support for a contact lens and a handle;
the support having an axis of symmetry extending vertically in use;
the handle extending radially from the support;
the handle being generally laminar and having upper and lower surfaces;
the support comprising an annular support body surrounding an axial drainage port, the support body having upper and lower surfaces;
wherein the upper surface of the support body is convex;
wherein one or both of the upper and lower surfaces of the handle may comprise a surface wholly or partially configured to facilitate use of the handle as a contact lens removal device by drawing of the surface across the in situ contact lens.

2. An applicator as claimed in claim 1, wherein the upper or lower surface of the handle comprises one or more of: one or more raised projections, relatively high friction regions or coatings, one or more regions or coatings having tack properties when in contact with a surface of a contact lens.

3. An applicator as claimed in claim 2, wherein one or both of the upper or lower surfaces of the handle wholly or partially comprises a relatively high friction material.

4. A contact lens applicator comprising
a support for a contact lens and a handle;
the support having an axis of symmetry extending vertically in use;
the handle extending radially from the support;
the handle being generally laminar and having upper and lower surfaces; the support comprising an annular support body surrounding an axial drainage port, the support body having upper and lower surfaces;
wherein the upper surface of the support body is convex; and
wherein upper and lower surfaces are configured so that the lower surface of the applicator may engage with an upper surface of a second identical applicator to form a stacked arrangement.

5. An applicator as claimed in any preceding claim, wherein the upper surface of the support is smooth, provided with raised features or projections or comprises one or more drainage channels.

6. An applicator as claimed in any preceding claim, wherein the handle extends radially from the support, perpendicular to the axis of the support.

7. An applicator as claimed in any of claims 1 to 5, wherein the handle extends radially in a direction downwardly or upwardly from the support in use.

8. An applicator as claimed in any preceding claim, wherein the handle has a first portion connected by a neck to the support, the handle extending from the axis, the first portion curving upwardly or extending at an angle to the horizontal and terminating in an alignment region adjoining a manual engagement region.

9. An applicator as claimed claim 8, wherein the angle is from 30° to 60°, preferably 45° to 60 °.

10. An applicator as claimed in claim 8 or 9, wherein the manual engagement portion of the handle extends radially outwardly from the alignment portion.

11. An applicator as claimed in any of claims 8 to 10, wherein the manual engagement portion comprises one or more raised projections.

12. An applicator as claimed in any preceding claim, wherein one or both of the upper and lower surfaces of the handle have a peripheral projection, beading or flange.

13. An applicator as claimed in any preceding claim, composed of polycaprolactone, polylactic acid or a copolymer or blend thereof.

14. An assembly comprising a plurality of applicators as claimed in any preceding claim joined by frangible connections to form a strip of applicators.

15. A kit comprising a plurality of applicators as claimed in any preceding claim, wherein the handles have two or more different lengths.
